# EUROPEAN PATENT SPECIFICATION

(11) **EP 2 032 184 B1**
(45) Date of publication and mention of the grant of the patent: **28.07.2010**
(21) Application number: 07762268.6
(22) Date of filing: 21.05.2007
(51) Int. Cl.: A61M 1/00

(54) **REFLUX CONTROL IN MICROSURGICAL SYSTEM**
RÜCKFLUSSSTEUERUNG IN EINEM MIKROCHIRURGISCHEN SYSTEM
CONTRÔLE DU REFLUX DANS UN SYSTÈME MICROCHIRURGICAL

(30) Priority: 23.06.2006 US 474190
(43) Date of publication of application: 11.03.2009
(73) Proprietor: Alcon, Inc., 6331 Hünenberg (CH)
(72) Inventor: HOPKINS, Mark A., Mission Viejo, California 92691 (US); GAO, Shawn X., Irvine, California 92629 (US)
(74) Representative: Moore, Barry
(86) International application number: PCT/US2007/069361
(87) International publication number: WO 2007/149667

(56) References cited:
- WO-A1-94/18894
- WO-A2-2007/001503
- US-A- 5 584 824
- US-A1- 2007 060 888
- US-A1- 2007 135 760
- US-B1- 6 599 271
- US-B2- 6 740 074
- US-B2- 7 143 782
- US-B2- 7 335 217

## Description

### Field of the Invention

The present invention generally pertains to controlling reflux in microsurgical systems and more particularly to controlling reflux in ophthalmic microsurgical systems.

### Description of the Related Art

During small incision surgery, and particularly during ophthalmic surgery, small probes are inserted into the operative site to cut, remove, or otherwise manipulate tissue. During these surgical procedures, fluid is typically infused into the eye, and the infusion fluid and tissue are aspirated from the surgical site. These probes have small orifices that are easily clogged with tissue. Such clogging is typically referred to as "occlusion", "tip occlusion", or "port occlusion". The process of clearing such occlusions is typically referred to as "reflux".

More generally, reflux is the ability to reverse the direction of the aspiration flow in a surgical system. Reflux may also be used for visualization of the surgical site (e.g. by moving blood and other tissue away from a particular point of interest).

A traditional method of reflux is to create a backpressure pulse of fluid that travels through the aspiration circuit to the tip or port of the probe to clear the incarcerated tissue. A hammer or valve is used to pinch a silicone tube to create a positive pressure pulse. This approach has no ability to control the reflux pressure profile. Therefore, a need continues to exist for an improved method of controlling reflux in a microsurgical system.

WO 9418894 describes a method and apparatus for minimally invasive tissue removal and contemplates a system including a tissue cutting tool having a motor driven cutting blade reciprocating within a percutaneously introduceable cannula. In accordance with one method, the frequency of reciprocation of the cutting blade is tuned to a characteristic frequency of the target tissue to be excised. Another aspect includes feedback control of both aspiration and irrigation circuits of the system, coupled with user settable inputs so the surgeon can control the system to "tease" tissue into the cutting opening. Controllable valves and pressure transducers allow the operator to set and maintain the aspiration vacuum and irrigation pressure at optimum levels.

US 6 740 074 describes a surgical system having a cassette with an aspirant collection chamber and an aspiration vent line that draws fluid from the aspirant collection chamber. The pressure within the collection chamber is maintained near ambient so that when the aspiration vent line is open, fluid flows from the collection chamber and into the aspiration line.

US 6 599 271 describes a device for preventing post occlusion flow surges during eye surgery which includes an enclosure defining an inlet and an outlet. The enclosure further defines a flow passage between the inlet and the outlet. The enclosure further defines a storage area for collecting material restrained by a filter structure.

### Summary of the Invention

The present invention provides an improved apparatus for controlling reflux in a microsurgical system. Accordingly there is provided an apparatus as provided in claim 1. Advantageous embodiments are provided in the dependent claims.

### Brief Description of the Drawings

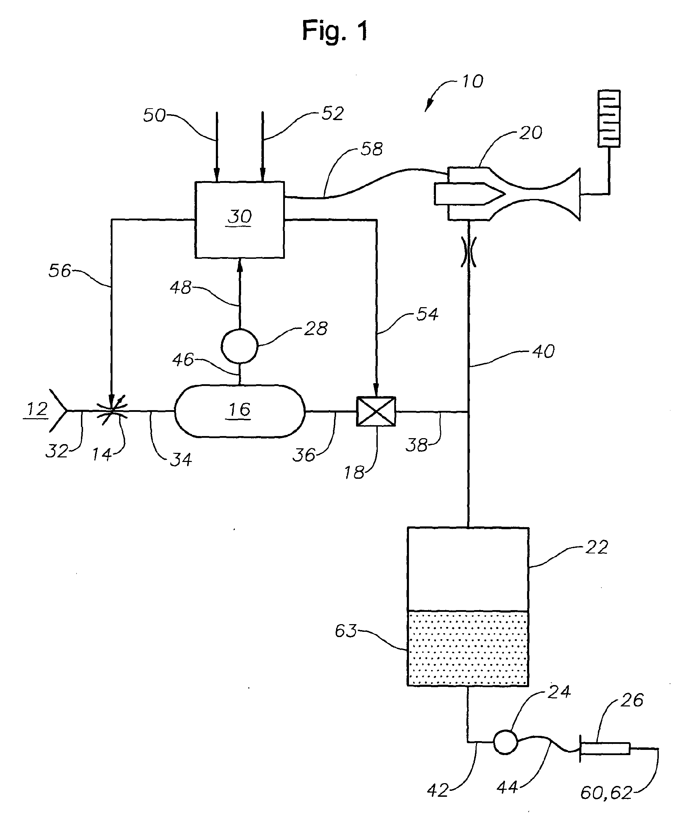

For a more complete understanding of the present invention, and for further objects and advantages thereof, reference is made to the following description taken in conjunction with the accompanying drawing, in which Figure 1 is a schematic diagram illustrating an aspiration circuit of a microsurgical system.

### Detailed Description of the Preferred Embodiments

The preferred embodiment of the present invention and its advantages is best understood by referring to Figure 1 of the drawings. Microsurgical system 10 preferably includes a pressurized gas source 12, a proportional valve 14, an accumulator 16, an isolation valve 18, a vacuum generator 20, an aspiration chamber 22, an aspiration port 24, a surgical device 26, a pressure transducer 28, and a computer or microprocessor 30. The various components of system 10 are fluidly coupled via fluid lines 32, 34, 36, 38, 40, 42, 44, and 46. The various components of system 10 are electrically coupled via interfaces 48, 50, 52, 54, 56, and 58. Valve 14 is preferably a proportional solenoid valve. Accumulator 16 preferably has a volume of about 15 cc. Valve 18 is preferably an "on/off" solenoid valve. Vacuum generator 20 may be any suitable device for generating vacuum but is preferably a vacuum chip or a venturi chip that generates vacuum. Surgical device 26 may be any surgical device that aspirates tissue but is preferably an ophthalmic surgical device such as a phacoemulsification probe, a vitrectomy probe, or an aspiration probe. Surgical device 26 has a tip 60 with a port 62 that is fluidly coupled to fluid line 44. Pressure transducer 28 may be any suitable device for directly or indirectly measuring pressure and vacuum. Microprocessor 30 is capable of implementing feedback control, and preferably PID control.

The aspiration circuit of Figure 1 enables improved control of reflux in microsurgical system 10. During normal operation of surgical device 26, fluid and/or tissue 63 are aspirated from port 62 into aspiration chamber 22 via vacuum supplied by vacuum generator 20. However, during a reflux operation of microsurgical system 10, microprocessor 30 sends a signal via interface 58 to turn off vacuum generator 20.

A user may input a setpoint for the desired pressure in accumulator 16 via interface 50. A user may also input whether a steady state reflux pressure or a pulsed reflux pressure is desired via interface 52. Alternatively, microprocessor 30 may provide a pre-defined reflux pressure profile for accumulator 16. Pressure transducer 46 measures the actual pressure within accumulator 16 and provides a corresponding signal to microprocessor 30 via interface 48. Microprocessor 30 compares the signal provided by pressure transducer 46 to the currently desired pressure for accumulator 16 and then adjusts proportional valve 14 via a signal over interface 56 so as to keep the measured reflux pressure of accumulator 16 at or near the desired reflux pressure.

When a steady state reflux pressure is commanded, microcontroller 30 maintains isolation valve 18 in an open position via a signal over interface 54. Microcontroller 30 then controls the reflux pressure within accumulator 16, aspiration chamber 22, and port 62 of surgical device 26 as described above. Since aspiration port 24 is located at the bottom of aspiration chamber 22, aspiration chamber 22 functions as a reservoir to provide sustained reflux, if necessary.

When a pulsed reflux pressure is commanded, microprocessor 30 momentarily closes isolation valve 18. Microprocessor 30 regulates the actual pressure within accumulator 16 as described above to create a "pre-charge" reflux pressure. Microprocessor 30 then closes proportional valve 14, opens isolation valve 18 to discharge the pre-charge reflux pressure in accumulator 16, and then re-closes isolation valve 18. In this manner, microprocessor 30 generates a pressure pulse that travels to aspiration chamber 22 and port 62 of surgical device 26. Such a pressure pulse is fully repeatable and programmable based upon the pre-defined reflux pressure profile stored in microprocessor 30.

Accumulator 16 also functions as a safety device. Once proportional valve 14 is closed, the maximum reflux pressure delivered to aspiration chamber 22 and port 62 is limited by the volume of accumulator 16 and the pre-charge reflux pressure.

The present invention is illustrated herein by example, and various modifications may be made by a person of ordinary skill in the art. For example, while the present invention is described above relative to reflux control in an ophthalmic microsurgical system, it is also applicable to other microsurgical systems.

It is believed that the operation and construction of the present invention will be apparent from the foregoing description. While the apparatus and methods shown or described above have been characterized as being preferred, various changes and modifications may be made therein without departing from the scope of the invention as defined in the following claims.

## Claims

1. Apparatus for controlling reflux in a microsurgical system (10), comprising:
a pressurized gas source (12);
an aspiration chamber (22) fluidly coupled to said pressurized gas source (12) and containing a fluid disposed therein;
a first valve (14) fluidly coupled to said pressurized gas source (12) and said aspiration chamber (22);
a second valve (18) fluidly coupled to said pressurized gas source (12) and said aspiration chamber (22);
an accumulator (16) fluidly coupled to said pressurized gas source (12) and said aspiration chamber (22) between said first valve (14) and said second valve (18);
a pressure transducer (28) fluidly coupled to said accumulator (16); and
a computer (30) electrically coupled to said first valve (14), said second valve (18), and said pressure transducer (28);
whereby said apparatus creates a reflux pressure pulse in said aspiration chamber (22) by said computer (30) maintaining said first valve (14) in an open state and said second valve (18) in a closed state, allowing pressurized gas to flow from said pressurized gas source (12) through said first valve (14) to form a pre-charge reflux pressure in said accumulator (16), closing said first valve (14), opening said second valve (18) to discharge said pre-charge reflux pressure into said aspiration chamber (22), and re-closing said second valve (18).

2. The apparatus of claim 1 wherein said first valve (14) is a proportional valve.

3. The apparatus of claim 2 wherein said formation of said pre-charge reflux pressure comprises:
said pressure transducer (28) determining an actual pressure within said accumulator (16) and providing a first signal corresponding to said actual pressure to said computer (30);
said computer (30) comparing said actual pressure to a desired pressure within said accumulator (16); and
said computer (30) providing a second signal to adjust said proportional valve (14) in response to said comparison of said actual pressure to said desired pressure.

4. The apparatus of claim 1 wherein said computer (30) creates multiple ones of said reflux pressure pulses in a repetitive manner.

5. The apparatus of claim 4 wherein said creation of said multiple reflux pressure pulses is performed according to a pre-defined profile in said computer (30).

6. The apparatus of claim 1 further comprising a surgical device (26) having a tip (60) with an open port (62) fluidly coupled to said aspiration chamber (22).

## Patentansprüche

1. Vorrichtung zum Steuern des Rückflusses in einem mikrochirurgischen System (10), mit:
einer Druckgasquelle (12);
einer Absaugkammer (22), die fluidisch mit der Druckgasquelle (12) gekoppelt ist und ein Fluid, das in ihr vorgesehen ist, enthält;
einem ersten Ventil (14), das fluidisch mit der Druckgasquelle (12) und der Absaugkammer (22) gekoppelt ist;
einem zweiten Ventil (18), das fluidisch mit der Druckgasquelle (12) und der Absaugkammer (22) gekoppelt ist;
einem Druckspeicher (16), der zwischen dem ersten Ventil (14) und dem zweiten Ventil (18) fluidisch mit der Druckgasquelle (12) und der Absaugkammer (22) gekoppelt ist;
einem Druckwandler (28), der fluidisch mit dem Druckspeicher (16) gekoppelt ist;
einem Computer (30), der elektrisch mit dem ersten Ventil (14), dem zweiten Ventil (18) und dem Druckwandler (28) gekoppelt ist;
wobei die Vorrichtung durch den Computer (30) einen Rückfluss-Druckimpuls in der Absaugkammer (22) erzeugt, der das erste Ventil (14) in einem offenen Zustand und das zweite Ventil (18) in einem geschlossenen Zustand hält,
wodurch Druckgas von der Druckgasquelle (12) durch das erste Ventil (14) fließen kann, um einen Vorlade-Rückflussdruck im Druckspeicher (16) zu bilden, wodurch das erste Ventil (14) schließt, das zweite Ventil (18) zum Entladen des Vorlade-Rückflussdruckes in die Absaugkammer (22) öffnet, und das zweite Ventil (18) erneut schließt.

2. Vorrichtung nach Anspruch 1, bei der das erste Ventil (14) ein Proportionalvenil ist.

3. Vorrichtung nach Anspruch 2, bei der die Bildung des Vorlade-Rückflussdruckes aufweist:
der Druckwandler (28) bestimmt den tatsächlichen Druck im Druckspeicher (16) und liefert ein erstes Signal entsprechend dem tatsächlichen Druck an den Computer (30);
der Computer (30) vergleicht den tatsächlichen Druck mit einem gewünschten Druck im Druckspeicher (16); und
der Computer (30) stellt ein zweites Signal bereit, um das Proportionalventil (14) als Antwort auf den Vergleich des tatsächlichen mit dem gewünschten Druck einzustellen.

4. Vorrichtung nach Anspruch 1, bei der der Computer (30) mehrere Rückflussdruckimpulse auf eine sich wiederholende Weise erzeugt.

5. Vorrichtung nach Anspruch 4, bei der die Erzeugung der mehreren Rückflussdruckimpulse gemäß einem vordefinierten Profil im Computer (30) erfolgt.

6. Vorrichtung nach Anspruch 1, die ferner ein chirurgisches Gerät (26) aufweist, das eine Spitze (60) mit einem offenen Port (62) hat, der fluidisch mit der Absaugkammer (22) gekoppelt ist.

## Revendications

1. Appareil de commande de reflux d'un système microchirurgical (10), comprenant :
une source de gaz sous pression (12) ;
une chambre d'aspiration (22) couplée de manière fluide à ladite source de gaz sous pression (12) et contenant un fluide placé dans celle-ci ;
une première vanne (14) couplée de manière fluide à ladite source de gaz sous pression (12) et ladite chambre d'aspiration (22) ;
une seconde vanne (18) couplée de manière fluide à ladite source de gaz sous pression (12) et ladite chambre d'aspiration (22) ;
un accumulateur (16) couplé de manière fluide à ladite source de gaz sous pression (12) et ladite chambre d'aspiration (22) entre ladite première vanne (14) et ladite seconde vanne (18) ;
un transducteur de pression (28) couplé de manière fluide audit accumulateur (16) ; et
un ordinateur (30) couplé électriquement à ladite première vanne (14), ladite seconde vanne (18), et ledit transducteur de pression (28) ;
de sorte que l'appareil produit une impulsion de pression de reflux dans ladite chambre d'aspiration (22) par ledit ordinateur (30) maintenant ladite première vanne (14) dans un état ouvert et ladite seconde vanne (18) dans un état fermé, permettant à un gaz sous pression de s'écouler depuis ladite source de gaz sous pression (12) à travers ladite première vanne (14) pour former une pression de reflux de précharge dans ledit accumulateur (16), fermant ladite première vanne (14), ouvrant ladite seconde vanne (18) afin d'évacuer ladite pression de reflux de précharge dans ladite chambre d'aspiration (22), et de refermer ladite seconde vanne (18).

2. Appareil selon la revendication 1, dans lequel ladite première vanne (14) est une vanne proportionnelle.

3. Appareil selon la revendication 2, dans lequel ladite formation de ladite pression de reflux de précharge comprend :
ledit transducteur de pression (28) déterminant une pression effective dans ledit accumulateur (16) et fournissant un premier signal correspondant à ladite pression effective audit ordinateur (30) ;
ledit ordinateur (30) comparant ladite pression effective à une pression souhaitée dans ledit accumulateur (16) ; et
ledit ordinateur (30) fournissant un second signal pour ajuster ladite vanne proportionnelle (14) en réponse à ladite comparaison de ladite pression effective à ladite pression souhaitée.

4. Appareil selon la revendication 1, dans lequel ledit ordinateur (30) crée de multiples impulsions de pression de reflux d'une manière répétitive.

5. Appareil selon la revendication 4, dans lequel ladite création desdites multiples impulsions de pression de reflux est réalisée selon un profil prédéfini dans ledit ordinateur (30).

6. Appareil selon la revendication 1 comprenant en outre un dispositif chirurgical (26) comprenant une extrémité (60) ayant une ouverture en position ouverte (62) couplée de manière fluide à ladite chambre d'aspiration (22).
